# EUROPÄISCHE PATENTSCHRIFT

(11) **EP 0 684 228 B1**
(45) Veröffentlichungstag und Bekanntmachung des Hinweises auf die Patenterteilung: **22.04.1998**
(21) Anmeldenummer: 95107323.8
(22) Anmeldetag: 15.05.1995
(51) Int. Cl.: C07C 303/22, C07C 309/40

(54) **Verfahren zur Herstellung von 4,4'-Dinitrostilben-2,2'-disulfonsäure und ihren Salzen**
Method for the preparation of 4,4'-dinitrostilbene-2,2'-disulphonic acid and its salts
Procédé pour la préparation de l'acide 4,4'-dinitrostilbène-2,2'-disulfonique et ses sels

(30) Priorität: 26.05.1994 DE 4418305
(43) Veröffentlichungstag der Anmeldung: 29.11.1995
(73) Patentinhaber: BAYER AG, 51368 Leverkusen (DE)
(72) Erfinder: Schomäcker, Reinhard, Dr., D-51381 Leverkusen (DE); Waldmann, Helmut, Dr., D-51373 Leverkusen (DE); Traenckner, Hans-Joachim, Dr., D-51375 Leverkusen (DE)

(56) Entgegenhaltungen:
- EP-A- 0 026 154
- EP-A- 0 332 137
- EP-A- 0 641 773

## Beschreibung

Die Erfindung betrifft ein Verfahren zur Herstellung von 4.4'-Dinitrostilben-2.2'-disulfonsäure (nachfolgend: "DNS") und ihren Salzen durch Oxidation von 4-Nitrotoluol-2-sulfonsäure (nachfolgend: "NTS") mit Sauerstoff in einem alkalischen Medium. Das Verfahren zeichnet sich durch eine elegante Isolierung des gewünschten Produkts aus.

DNS ist ein wichtiges Zwischenprodukt für die Herstellung optischer Aufheller; sie wird daher in großem Umfang hergestellt.

Verfahren zur Herstellung von DNS durch Oxidation von NTS mit Sauerstoff in wäßrig alkalischem Medium sind bekannt (DD 240 200, DE-OS 3 409 171 und 3 519 552, EP-A 305 648). Problematisch ist dabei die schlechte Löslichkeit von NTS in Natron- und Kalilauge. Sofern überhaupt sehr gute Ausbeuten erzielt werden, arbeiten diese Verfahren mit hoher Verdünnung, langen Reaktionszeiten oder unter Druck oder sie sind unter Berücksichtigung der anfallenden Abwässer unwirtschaftlich und konnten deshalb nicht befriedigen.

Es ist auch bereits vorgeschlagen worden, in dipolaren aprotischen Lösungsmitteln zu arbeiten (EP-A 26 154 und 332 137). Auf der einen Seite ist es wünschenswert, NTS als wäßrige Lösung oder in wasserfeuchtem Zustand einzusetzen, also auf eine vollständige Trocknung bei der NTS-Herstellung zu verzichten. Auf der anderen Seite haben eigene Versuche ergeben, daß sich die Gegenwart von Wasser beim Arbeiten in dipolaren aprotischen Lösungsmitteln auf die Ausbeute stark negativ auswirkt.

Es wurde nun gefunden, daß die Verwendung einer wäßrigen Lösung bestimmer Etheralkohole eine günstige Reaktionsführung bei hoher Ausbeute und eine stark vereinfachte Aufarbeitung ermöglicht.

Gegenstand der Erfindung ist also ein Verfahren zur Herstellung von Verbindungen der Formel 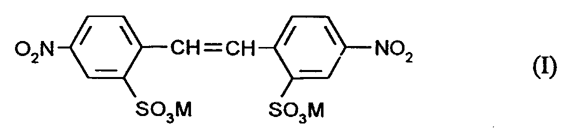 worin M für Wasserstoff, Natrium oder Kalium steht, durch Oxidation von 4-Nitrotoluol-2-sulfonsäure mit Sauerstoff in einem Lösungsmittel bei Temperaturen von 30 bis 80, vorzugsweise 35 bis 55°C, in gegenwart einer Base,
dadurch gekennzeichnet, daß man als Lösungsmittel ein Gemisch aus
a) 50 bis 70 Gew.-% Wasser und
b) 30 bis 50 Gew.-% Etheralkohol verwendet,
wobei sich die Prozentangaben auf die Summe (a+b) beziehen und der Etheralkohol der Formel

CᵢH₂ᵢ₊₁(OCH₂CH₂)ⱼ-OH (II)

entspricht, wobei
- i: 2 bis 10, vorzugsweise 2 bis 5, und
- j: 1 bis 6, vorzugsweise 1 bis 3 bedeuten,
und die Aufarbeitung des Reaktions gemisches durch Phasentrennung in gegenwart eines unpolaren organischen Lösungsmittels bei einer Temperatur von 60 bis 100°C erfolgt.

Der Begriff "Sauerstoff' im Sinne der Erfindung umfaßt auch Sauerstoff-Mischungen mit anderen Gasen, wie Kohlendioxid oder Stickstoff. Die wirtschaftlichste Form ist die atmosphärische Luft. Es ist vorteilhaft, den Sauerstoff im Reaktionsgemisch möglichst fein zu verteilen, beispielsweise durch Verwendung geeigneter Düsen.

Die Reaktion wird in Gegenwart von Basen durchgeführt. Bevorzugte Basen sind die Alkalihydroxide, wobei Natrium- und Kaliumhydroxid besonders bevorzugt sind. Die Basenmenge kann sich innerhalb weiter Grenzen bewegen; sie hängt u.a. davon ab, ob man die NTS als freie Säure oder als Salz einsetzt. Da beim Einsatz der NTS als freie Säure für die Neutralisation der Sulfonsäuregruppe 1 Äquivalente Base verbraucht wird, benötigt man die Base in einer mehr als äquivalenten Menge. Bevorzugt wird die Base in einer Menge von 1.5 bis 8, vorzugsweise 2 bis 5 Äquivalenten pro Mol NTS (freie Säure) eingesetzt.

Die Ausführung des erfindungsgemäßen Verfahrens unter Zusatz eines Katalysators kann vorteilhaft sein, ist aber nicht unbedingt notwendig. Als Katalysatoren kommen insbesondere Verbindungen der Übergangsmetalle, beispielsweise von Co, Cr, Fe, Ni, Cu, Nb, Ta, Ru, vorzugweise Mn und V, zum Einsatz. Mögliche Einsatzformen dieser Metalle sind deren Salze anorganischer Säuren, beispielsweise die Metallfluoride, -chloride, -sulfate, -nitrate, -carbonate, -phosphate; die Metalloxide und Metallhydroxide; die Metallsalze organischer Säuren, beispielsweise die Metallacetate, -oxalate, -phenolate, -benzoate, -salicylate; Komplexe dieser Metalle beispielsweise mit Acetylaceton, N,N'-Disalicyliden-ethylendiamin, Ethylendiamin, Diethylentriamin, Triethylentetramin, Tetraphenylporphin und Phthalocyanin.

Die Katalysatoren können in Mengen von 0,1 bis 10 g, vorzugsweise 0,2 bis 1 g, jeweils pro kg Reaktionsmischung, verwendet werden.

Bevorzugte Etheralkohole II umfassen die C₁-C₁₀-Alkylether, insbesondere die Methyl-, Ethyl-, Propyl-, Butyl-, Pentyl- und Hexylether von Ethylenglykol, Diethylenglykol, Triethylenglykol, Quatroethylenglykol, Pentaethylenglykol und Hexaethylenglykol. Besonders bevorzugt sind die Ethyl-, Propyl- und Butylether von Di- und Triethylenglykol, insbesondere Ethylenglykolmonoethyl- und mono- butylether. Zu einer besonders bevorzugten Ausführungsform wird eine Mischung aus Diethylenglykolmonoethyl- und -monobutylether eingesetzt, die mit den anderen Komponenten des Reaktionsgemisches unter Reaktionsbedingungen einphasig, unter Aufarbeitungsbedingungen aber zweiphasig ist. So kann das für das erfindungsgemäße Verfahren einzusetzende Lösungsmittel beispielsweise aus
a) 50 bis 70 Gew.-% Wasser,
b)i) 10 bis 30 Gew.-% Diethylenglykolmonobutylether und
b)ii) Diethylenglykolmonoethylether bestehen, wobei sich die Mengen a), b)i) und b)ii) auf 100 Gew.-% ergänzen.

Beim erfindungsgemäßen Verfahren kann man also zum Beispiel so vorgehen, daß man eine Lösung von NTS in einer Mischung aus 5 bis 10 gew.-%iger Natronlauge oder Kalilauge und Etheralkohol bei 30 bis 80, vorzugsweise 35 bis 55°C mit Sauerstoff oder Luft begast, gegebenenfalls in Gegenwart eines Katalysators. Es ist manchmal empfehlenswert, am Anfang nur einen Bruchteil der NTS-Menge einzusetzen und den Rest, gegebenenfalls in Wasser oder Etheralkohol gelöst, im Laufe der Reaktion nachzudosieren. Das Fortschreiten der Reaktion läßt sich beispielsweise dünnschichtchromatographisch verfolgen Nach Beendigung der Reaktion kann man durch Zusatz von Säure, z.B. Schwefelsäure, neutralisieren. Es wurde gefunden, daß man, gegebenenfalls nach Neutralisation - je nach pH-Wert - die DNS selbst oder ihr Salz (vorzugsweise das Dinatriumsalz) direkt aus dem Reaktionsgemisch durch Kühlen auf 5 bis 30, vorzugsweise 10 bis 25°C zur Kristallisation bringen kann. Das kristallisierte Produkt kann dann auf an sich bekannte Weise, z.B. durch Filtration oder Zentrifugieren, von der Mutterlauge abgetrennt werden.

Durch die bei der Neutralisation entstehende Salzmenge wird die Trennung der Reaktionsmischung in zwei Phasen begünstigt. Es hat sich gezeigt, daß man die gewünschte Phasentrennung (von Reaktionsmischung oder Mutterlauge) weiter fördern kann, wenn man die Temperatur auf vorzugsweise 70 bis 80°C einstellt. In der wäßrigen Phase finden sich anorganische Salze und wasserlösliche Reaktionsnebenprodukte, in der organischen Phase nicht auskristallisierte DNS und gegebenenfalls noch Ausgangsprodukt. Weiter erleichtert wird die Phasentrennung durch die Zugabe unpolarer organischer Lösungsmittel. Zur Extraktion geeignete unpolare organische Lösungsmittel umfassen z.B. aliphatische, cycloaliphatische und aromatische C₅-C₁₈-Kohlenwasserstoffe wie n-Pentan, iso-Pentan, n-Hexan, Cyclohexan, n-Heptan, n-Oktan, iso-Oktan, n-Nonan, iso-Nonan, n-Dekan, n-Undekan, n-Dodekan, iso-Dodekan, n-Tridekan, n-Tetradekan, n-Pentadekan, n-Hexadekan, iso-Hexadekan, n-Heptadekan, n-Oktadekan, Benzol, C₁-C₉-Alkylbenzole wie Toluol, Xylol und Mesitylen. Die C₅-C₁₈-Kohlenwasserstoffe können auch durch Hydroxyl, Chlor, Brom, Fluor, Nitro, Hydroxy-C₁-C₄-alkyl oder C₁-C₄-Alkoxy 1- bis 3-fach, vorzugsweise einfach, substituiert sein. Derartige Lösungsmittel umfassen z.B. Chlorbenzol, Chlortoluol, 2-Ethylhexanol, Bis(hydroxymethyl)-cyclohexan und Anisol. Besonders bevorzugt sind aromatische Kohlenwasserstoffe; Toluol ist das bevorzugteste Extraktionsmittel.

Nach einer bevorzugten Ausführungsform erfolgt die Phasentrennung in Gegenwart der unpolaren organischen Lösungsmittel 70 bis 80°C.

Das Extraktionsmittel kann in einem Verhältnis zum Reaktionsgemisch in Gewichtsverhältnissen von 1:0.05 bis 1:1 zugesetzt werden. Diese Extraktion kann ein- oder mehrstufig im Gegenstrom oder im Kreuzstrom in geeigneten Apparaturen, wie Extraktionskolonnen oder Mixer-Settler-Apparaturen, durchgeführt werden. Aus der Mutterlauge können Etheralkohol und nicht kristallisierende Wertstoffe mit Wasser reextrahiert und in die Reaktion zurückgeführt werden; die organische Phase dieses Extraktionsschrittes enthält überwiegend das für die Extraktion des Endprodukts verwendete unpolare organische Lösungsmittel, das zurückgewonnen und ohne weitere Reinigung für weitere Extraktionen verwendet werden kann.

Man kann erfindungsgemäß auch Kristallisation und Extraktion vertauschen und zunächst aus dem Reaktionsgemisch die organischen Wertstoffe mit einem unpolaren organischen Lösungsmittel extrahieren und aus diesem Extrakt DNS oder ihr Salz auskristallisieren lassen.

Zur Aufarbeitung des Extraktes kann man also durch Abkühlen des Extraktes auf 5 bis 30, bevorzugt auf 10 bis 20°C, die DNS zur Kristallisation bringen. Die Mutterlauge wird anschließend auf 50 bis 100, bevorzugt 70 bis 80°C erwärmt und trennt sich in zwei Phasen. Zur Verbesserung der Trennung wird z.B. Toluol als Extraktionsmittel zugesetzt. Die wäßrige Phase, kann gegebenenfalls wiederholt, mit Toluol extrahiert werden.

Die organischen Phasen der Extraktionsschritte können dann vereinigt und auf Temperaturen von 10 bis 20°C abgekühlt werden. Bei dieser Temperatur lassen sich die Etheralkohole mit Wasser reextrahieren. Die Wassermenge wird dabei vorzugsweise so gewählt, daß die für die Reaktion benötigte Zusammensetzung der Wasser-/Etheralkohol-Mischung entsteht. Die Oberphase dieser Reextraktion kann ohne weitere Reinigung in die Extraktion der Mutterlauge zurückgeführt werden.

### Beispiele

### Beispiel 1

In einer kontinuierlich betriebenen Apparatur wurden in eine Kaskade aus vier 5-l Rührkesseln, von denen die beiden ersten auf 45°C und die beiden zweiten auf 55°C temperiert wurden, pro Stunde 1 000 g Wasser, 800 g Diethylenglykolmonoethylether, 200 g Diethylenglykolmonobutylether, 145 g NaOH und 200 g NTS eindosiert. Jeder Kessel wurde mit 25 l/h Luft begast. Der Produktstrom, der die Kaskade verließ, wurde mit 180 g/h konz. Schwefelsäure neutralisiert und bei 15°C zur Kristallisation gebracht. Die Mutterlauge wurde in einer auf 70°C temperierten Mixer-Settler-Apparatur mit 300 g/h Toluol versetzt. Die abgetrennte wäßrige Phase wurde in einer zweiten auf 75°C temperierten Mixer-Settler-Apparatur erneut mit 300 g/h Toluol extrahiert. Die wäßrige Phase wurde verworfen, die organischen Phasen aus beiden Extraktionen wurden vereinigt. Durch eine Extraktion mit 1000 g/h Wasser, die bei 15°C durchgeführt wurde, wurden die eingesetzten Etheralkohole und nicht kristallisierte Wertstoffe gewonnen und nach Abziehen von Toluolresten in die Kesselkaskade zurückgeführt. Die organische Phase, die im wesentlichen aus Toluol besteht, wurde in die Extraktionen der Mutterlauge zurückgeführt. Die Ausbeute dieses Verfahrens betrug >190 g/h DNS, d.h. >95 % d.Th.

### Beispiel 2

In einem 2-Liter-Rührkessel wurden 1 000 g Wasser, 800 g Diethylenglykolmonoethylether, 200 g Diethylenglykolmonobutylether und 120 g Natriumhydroxid gegeben und auf 45°C temperiert. Nach Erreichen der Reaktionstemperatur wurde der Reaktorinhalt mit 100 l/h Luft begast. Unter ständigem Begasen wurden beim Start der Reaktion 100 g 4-Nitrotoluol-2-sulfonsäure zugegeben. Über vier Stunden wurden weitere 100 g 4-Nitrotoluol-2-sulfonsäure und 25 g Natriumhydroxid nachgesetzt. Nach beendeter Zugabe, d.h. nach 4 Stunden wurde die Reaktionstemperatur auf 55 bis 57°C erhöht und der Ansatz für 3 Stunden ausreagieren lassen. Zur Produktisolierung wurde der Ansatz unter weiterer Sauerstoffbegasung mit konzentrierter Schwefelsäure neutralisiert. Danach wurde die Reaktionsmasse auf 75°C temperiert und mit 300 g Toluol versetzt. Bei dieser Temperatur erfolgte eine rasche Phasentrennung in eine wäßrige und eine organische Phase. Die wäßrige Phase wurde abgetrennt, erneut mit 300 g Toluol extrahiert und anschließend verworfen. Die vereinigten organischen Phasen wurden durch Abkühlen auf 10 bis 15°C zur Kristallisation gebracht. Aus Mutterlauge wurden bei 10 bis 15°C nicht kristallisiertes Produkt, Diethylenglykolmonoethylether und Diethylenglykolmonobutylether mit Wasser extrahiert und in die Reaktion zurückgeführt. Die Ausbeute an 4,4'-Dinitrostilben-2,2'-disulfonsäure wurde aus der Summe der isolierten Menge und dem per HPLC bestimmten Gehalt der Mutterlauge bestimmt. Die Ausbeute betrug >95 % der Theorie.

### Beispiel 3

In einer kontinuierlich betriebenen Apparatur wurden in eine Kaskade aus vier 5-l Rührkesseln, die auf 50°C temperiert wurde, pro Stunde 1 000 g Wasser, 800 g Diethylenglykolmonoethylether, 200 g Diethylenglykolmonobutylether, 145 g Natriumhydroxid und 200 g NTS eindosiert. Jeder Kessel wurde mit 25 1 Luft pro Stunde begast. Der Produktstrom, der die Kasskade verließ, wurde in einer auf 70°C temperierten Mixer-Settler-Apparatur mit 180 g/h konzentrierter Schwefelsäure neutralisiert und mit 300 g/h Toluol versetzt. Die abgetrennte wäßrige Phase wurde in einer zweiten auf 75°C temperierten Mixer-Settler-Apparatur erneut mit 300 g/h Toluol extrahiert. Die wäßrige Phase wurde verworfen, die organischen Phasen aus beiden Extraktionen wurden vereinigt und bei 15°C zur Kristallisation gebracht.. Aus der Mutterlauge wurden durch eine Extraktion mit 1000 g/h Wasser bei 15°C die eingesetzten Etheralkohole und nicht kristallisierte Wertstoffe gewonnen und nach Abziehen von Toluolresten in die Kesselkaskade zurückgeführt. Die organische Phase, die im wesentlichen aus Toluol besteht, wurde in die Extraktionen zurückgeführt. Die Ausbeute dieses Verfahren betrug >190 g/h an DNS, d.h. >95 % der Theorie.

## Patentansprüche

1. Verfahren zur Herstellung von Verbindungen der Formel 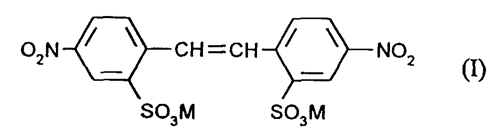 worin M für Wasserstoff, Natrium oder Kalium steht, durch Oxidation von 4-Nitrotoluol-2-sulfonsäure mit Sauerstoff in einem Lösungsmittel bei Temperaturen von 30 bis 80°C, in gegenwart einer Base, dadurch gekennzeichnet, daß man als Lösungsmittel ein Gemisch aus
a) 50 bis 70 Gew.-% Wasser und
b) 30 bis 50 Gew.-% Etheralkohol verwendet,
wobei sich die Prozentangaben auf die Summe (a+b) beziehen und der Etheralkohol der Formel
CᵢH₂ᵢ₊₁(OCH₂CH₂)ⱼ-OH (II)
entspricht, wobei
i 2 bis 10 und
j 1 bis 6 bedeuten,
und die Aufarbeitung des Reaktionsgemisches durch Phasentrennung in gegenwart eines unpolaren organischen Lösungsmittels bei einer Temperatur von 60 bis 100°C erfolgt.

2. Verfahren nach Anspruch 1, wobei
i 2 bis 5 und
j 1 bis 3 bedeuten.

3. Verfahren nach Anspruch 1, wobei der Etheralkohol Diethylenglykolmonoethylether und/oder Diethylenglykolmonobutylether ist.

4. Verfahren nach Anspruch 1, wobei das Lösungsmittel aus
a) 50 bis 70 Gew.-% Wasser,
b)i) 10 bis 30 Gew.-% Ethylenglykolmonobutylether und
b)ii) Ethylenglykolmonoethylether besteht,
wobei sich die Mengen a), b)i) und b)ii) auf 100 Gew.-% ergänzen.

5. Verfahren nach Ansprüchen 1 bis 4, wonach man die 4-Nitrostilbensulfonsäure oder ihr Salz bei einer Temperatur von 5 bis 30°C auskristallisieren läßt.

6. Verfahren nach Ansprüchen 1 bis 5, wonach man bei der Aufarbeitung des Reaktionsgemisches mit einem unpolaren organischen Lösunngsmittel extrahiert.

7. Verfahren nach Ansprüchen 1 bis 6, wonach man den Etheralkohol b) und gegebenenfalls eingesetztes unpolares organisches Lösungsmittel zurückgewinnt und erneut in die Reaktion einsetzt.

## Claims

1. Process for preparing compounds of the formula 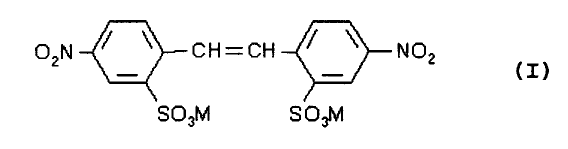 in which **M** represents hydrogen, sodium or potassium, by oxidation of 4-nitrotoluene-2-sulphonic acid with oxygen in a solvent at temperatures of from 30 to 80°C, in the presence of a base,
characterized in that the solvent used is a mixture of
a) from 50 to 70% by weight of water and
b) from 30 to 50% by weight of ether alcohol,
where the percentages implicated are based on the sum (a+b) and the ether alcohol has the formula
CᵢH₂ᵢ₊₁(OCH₂CH₂)ⱼ-OH (II),
where
i is from 2 to 10 and
j is from 1 to 6,
and the reaction mixture is worked up by phase separation in the presence of a nonpolar organic solvent at a temperature of from 60 to 100°C.

2. Process according to Claim 1, where
i is from 2 to 5 and
j is from 1 to 3.

3. Process according to Claim 1, where the ether alcohol is diethylene glycol monoethyl ether and/or diethylene glycol monobutyl ether.

4. Process according to Claim 1, where the solvent consists of
a) from 50 to 70% by weight of water,
b)i) from 10 to 30% by weight of ethylene glycol monobutyl ether and
b)ii) ethylene glycol monoethyl ether,
where the amounts a), b)i) and b)ii) add up to 100% by weight.

5. Process according to any of Claims 1 to 4, according to which the 4-nitrostilbenesulphonic acid or its salt is allowed to crystallize out at a temperature of from 5 to 30°C.

6. Process according to any of Claims 1 to 5, according to which extraction with a nonpolar organic solvent is carried out during the work-up of the reaction mixture.

7. Process according to any of Claims 1 to 6, according to which the ether alcohol b) and any nonpolar organic solvent used are recovered and again used in the reaction.

## Revendications

1. Procédé pour la préparation de composés répondant à la formule 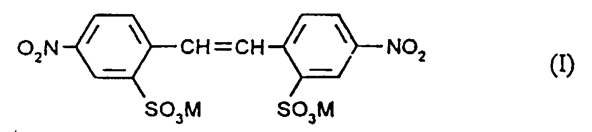 dans laquelle M représente un atome d'hydrogène, un atome de sodium ou un atome de potassium, par oxydation de l'acide 4-nitrotoluène-2-sulfonique avec de l'oxygène dans un solvant, à des températures de 30 à 80°C, en présence d'une base,
caractérisé en ce qu'on utilise comme solvant un mélange constitué par
a) à concurrence de 50 à 70% en poids, de l'eau et
b) à concurrence de 30 à 50% en poids, un éther-alcool,
les indications en pour cent se rapportant à la somme (a+b) et l'éther-alcool répondant à la formule
CᵢH₂ᵢ₊₁ (OCH₂CH₂)ⱼ-OH (II)
dans laquelle
i représente une valeur de 2 à 10 et
j représente une valeur de 1 à 6,
et dans lequel le traitement du mélange réactionnel a lieu par séparation de phases en présence d'un solvant organique apolaire, à une température de 60 à 100°C.

2. Procédé selon la revendication 1, dans lequel
i représente une valeur de 2 à 5 et
j représente une valeur de 1 à 3.

3. Procédé selon la revendication 1, dans lequel l'éther-alcool est l'éther monoéthylique de diéthylèneglycol et/ou l'éther monobutylique de diéthylèneglycol.

4. Procédé selon la revendication 1, dans lequel le solvant est constitué par
a) à concurrence de 50 à 70% en poids, de l'eau
b)i) à concurrence de 10 à 30% en poids, l'éther monobutylique d'éthylèneglycol et
b)ii) l'éther monoéthylique d'éthylèneglycol,
les quantités a), b)i) et b)ii) se complétant pour donner 100%.

5. Procédé selon les revendications 1 à 4, dans lequel on laisse se cristalliser l'acide 4-nitrostilbènesulfonique ou son sel à une température de 5 à 30°C.

6. Procédé selon les revendications 1 à 5, dans lequel, lors du traitement du mélange réactionnel, on procède à une extraction dans un solvant organique apolaire.

7. Procédé selon les revendications 1 à 6, dans lequel on récupère l'éther-alcool b) et éventuellement le solvant organique apolaire mis en oeuvre et on les remet en oeuvre dans la réaction.
